(19) Europäisches Patentamt
European Patent Office
Office européen des brevets

(11) EP 0 392 837 B1

(12) **EUROPEAN PATENT SPECIFICATION**

(45) Date of publication and mention
of the grant of the patent:
**31.01.1996 Bulletin 1996/05**

(51) Int. Cl.⁶: **A61B 17/39**

(21) Application number: **90303939.4**

(22) Date of filing: **11.04.1990**

(54) **Apparatus for thermally destroying tissue**

Vorrichtung zur thermischen Zerstörung von Geweben

Appareil de destruction thermique des tissus

(84) Designated Contracting States:
**AT BE CH DE DK ES FR GB GR IT LI LU NL SE**

(30) Priority: **13.04.1989 US 337356**

(43) Date of publication of application:
**17.10.1990 Bulletin 1990/42**

(73) Proprietors:
• **Geddes, Leslie Alexander**
**West Lafayette, Indiana 47906 (US)**
• **Hinds, Marvin Harold**
**Marion, Indiana 46953 (US)**
• **Bourland, Joe Dan**
**West Lafayette, Indiana 47906 (US)**
• **Voorhees, William Delano**
**West Lafayette,Indiana 47906 (US)**

(72) Inventors:
• **Geddes, Leslie Alexander**
**West Lafayette, Indiana 47906 (US)**
• **Hinds, Marvin Harold**
**Marion, Indiana 46953 (US)**
• **Bourland, Joe Dan**
**West Lafayette, Indiana 47906 (US)**
• **Voorhees, William Delano**
**West Lafayette,Indiana 47906 (US)**

(74) Representative: **Johnston, Kenneth Graham**
**Woodford Green Essex, IG8 OTU (GB)**

(56) References cited:
EP-A- 0 182 689          EP-A- 0 222 137
FR-A- 2 501 034          US-A- 4 696 668

• **D1 = US - A - 4 676 258**

## Description

This invention relates to apparatus for thermally destroying or coagulating living tissue.

Gall bladder problems are very prevalent and the need for preventing the recurrence of gallstones is significant.

The foregoing problems are solved and a technical advance is achieved by apparatus for thermally destroying or coagulating the layer of or on living tissue such as an organ. The thermal coagulation of the mucosal layer of a gall bladder will reduce, and may eliminate, the recurrence of gallstones.

U.S.-A-4,676,258 discloses apparatus as defined in the preamble of claim 1. During the operation of the R.F. emitter arrangement, liquid is circulated through the balloon to act as a coolant.

According to the present invention there is provided apparatus as defined in claim 1.

The extendible arrangement can take several forms, in one of which the distal end of the elongated member may be found in expandable strips which can expand to contact the wall of a gall bladder. The current-emitting electrode is internally affixed to the distal tip of the member and operated to expand the strips radially and hold the electrode in the centre of the gall bladder. The expanded strips engage and make contact with the inner mucosal layer of the gall bladder. Experiments with this conductive electrode catheter indicated that an electrode temperature of 72°C was needed to maintain the mucosal layer at 50°C while the outer wall temperature was approximately 41°C. The radiofrequency generator utilized in these experiments had a tentative frequency of two megahertz with signal amplitude ranging between 0-2 amps rms of radiofrequency current.

In such an arrangement the bile in the gall bladder is used to conduct the RF energy to the said wall. Some form of seal, additional to the presence of the apparatus, could be required to enclose and maintain the bile within the bladder.

If the possibility of gall bladder rupture is a problem, then the leakage of bile from such a rupture could be prevented by providing a balloon about the said distal end. The latter would be expanded by inserting liquid therein to expand the balloon to the normal boundary of the gall bladder.

If bile leakage were not a problem then a balloon could be partially expanded by partially filling it with liquid. Some bile could be retained thereabout in the bladder.

In both of these latter embodiments, the balloon would serve the function of both locating the emitter and containing liquid within the bladder. The liquid within the bladder can have any resistivity which is consistent with the objective of assisting in the destruction or coagulation (burning) of the inner wall of the bladder. It may also be made radio-opaque.

The balloon may have more than one compartment so that different fluids can be forced therein, the fluid of higher resistivity being forced into the outer compartment.

In the embodiments of the invention, the inner wall of the gall bladder is preferably coagulated at temperatures and times within the preferred range of 44.5°C at 1 hour; 47°C at 15 minutes; 50°C at 6 minutes; and 60°C at 1.2 minutes.

FIG.1 depicts apparatus inserted in the body of the gall bladder for thermally destroying the mucosal layer thereof;
FIG.2 depicts a cross-sectional view of the elongated member of the apparatus of FIG.1;
FIG.3 depicts a spherical model of a gall bladder;
FIG.5 depicts the power deposition at a radial distance from a central electrode placed in the spherical model; and

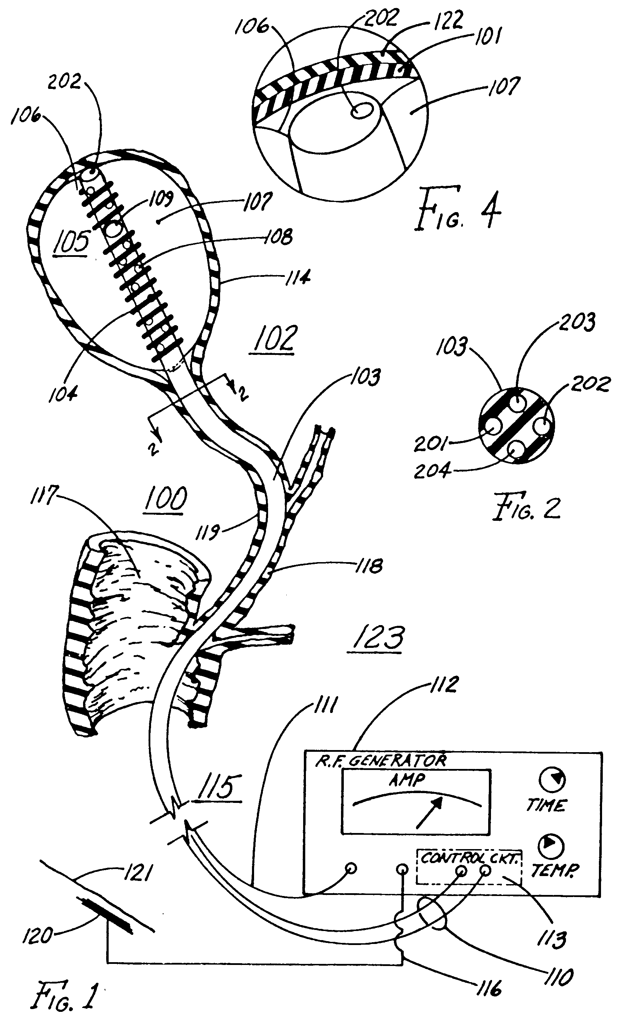

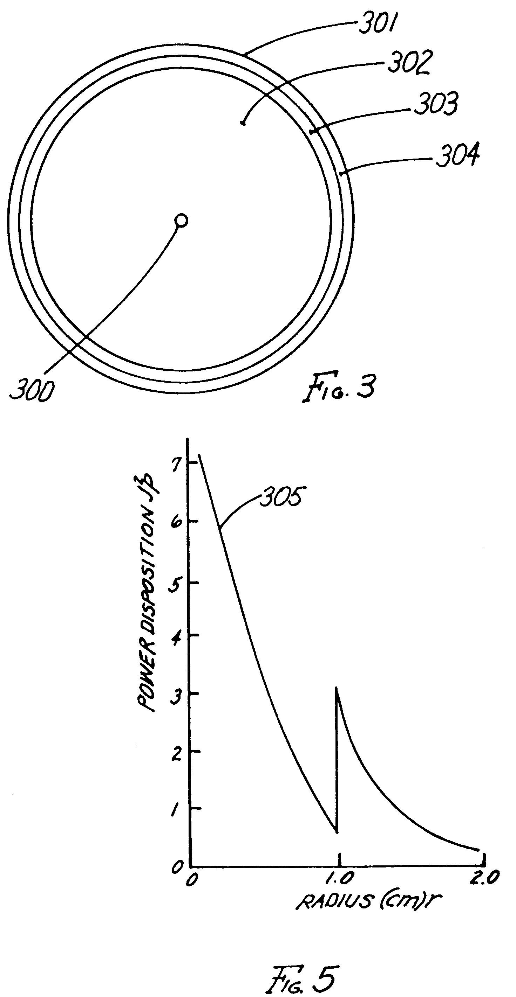

FIG.4 depicts an enlarged view of the distal end of the elongated member of the apparatus of FIG. 1.

Depicted in FIG.1 is illustrative apparatus 123 including a catheter 100 having a distal end 105 that is inserted into an organ such as body 114 of gall bladder 102 for thermally coagulating and/or destroying the inner mucosal layer 101 of the gall bladder. The distal end of the catheter is endoscopically introduced into the body of the gall bladder by a retrograde route through the duodenum 117, common bile duct 118, and cystic duct 119. Catheter 100 includes an elongated member 103 with a current-emitting electrode 104 positioned about the distal end thereof for emitting radiofrequency current to the mucosal layer of the gall bladder. Also positioned about the distal end of the elongated member and surrounding the current-emitting electrode is capacitive balloon electrode 106, which is expandable for making physical and electrical contact with the mucosal layer of the gall bladder.

The conductive balloon is expanded by forcing liquid 107 therein. The resistivity of the liquid is such as to concentrate the deposition of radiofrequency power in the mucosal layer. The mucosal layer 101 of gall bladder 102 has a relatively high level of electrical resistivity approximating, for example, 500 ohm-cm. The electrical resistivity of the electrolyte solution used in one embodiment is at a much lower level, for example, about 10 ohm-cm. In another embodiment of the invention the bile of the gall bladder is used and that has a resistivity of approximately 70 ohm-cm. The change in resistivity from 10 ohm-cm to 500 ohm-cm represents a significant gradient in the order of one and a half orders of magnitude. The radiofrequency heating uses the change in electrical resistivity from the liquid or bile to the mucosal layer at the interface thereof. The object is to concentrate power deposition in the inner mucosal layer of the gall bladder, namely in the media of higher electrical resistivity. It is this power deposition as a function of distance from the current-emitting electrode that produces selective heating.

A spherical model of a gall bladder is depicted in FIG.3 to illustrate the selective heating. A current-emitting point electrode 300 is positioned at the centre of a spherical gall bladder 301 of 1.0cm inner radius filled with bile 302 having a resistivity of 70 ohm-cm. Beyond 1cm is the inner mucosal layer 303 and outer gall bladder wall 304 of resistivity approximating 500 ohm-cm. Beyond the outer gall bladder wall is the liver (not shown) with approximately the same resistivity. The deposited power density as a function of radial distance from the electrode is illustrated by curve 305 depicted in FIG.5. The deposited power density at any point on curve 305 is $J^2p$, where J is the current density (current I divided by area) and p is the electrical resistivity. At any radius r, the current density $J=I(4\pi r^2)$. Current density J decreases as the inverse square of the distance from the electrode in the centre of the sphere. From the central electrode, the radiofrequency current first encounters the low-resistivity bile and then the high-resistivity bladder wall and extrabladder tissues. It is the deposited power that produces heating. At a distance beyond the bile of the gallbladder (r>1.0cm), the current density continues to decrease, but the resistivity rises to a high level such as 500 ohm-cm. Consequently, there is a peak in power deposition in the inner mucosal layer 303 of the gallbladder where the resistivity changes from 70 ohm-cm to 500 ohm-cm. As a result, there is a concentration of deposited power in and selective heating of the mucosal layer at the bile-gallbladder interface.

In the embodiment shown in FIG.1, balloon electrode 106 is expanded with an electrolyte solution 107 having a resistivity lower than that of the gallbladder bile. This selectively concentrates the power deposition and heating in the mucosal layer. In this embodiment, the electrolyte solution used to expand the balloon comprises a solution of 5% saline with potassium iodide added to provide radio-opacity. The addition of 5 grams of potassium iodide to 100 ml of 5% saline reduces the resistivity of the latter by 25%. The resistivity of the resulting mixture is 10 ohm-cm at 37°C. The resistivity gradient at the electrolyte solution-mucosal layer interface is now 10/500, rather than 70/500 when bile is used.

Depicted in FIG.2 is a cross-sectional view of the elongated member 103 of the catheter. The elongated member includes a plurality of lumens 201-204 that extend the entire longitudinal length of the member. Filling lumen 201 is for transporting the electrolyte solution from the proximal end 115 of the catheter to the distal end 105. As shown in FIG.1, a plurality of side ports 108 about the distal end connected to filling lumen 201 facilitates the expansion of capacitive balloon 106. The balloon is attached to the distal end of the catheter and expands with the electrolyte solution to make electrical and physical contact with mucosal layer 101 of the gall bladder. The balloon electrode also contains the electrolyte solution and prevents caustic solutions from causing possible undesired injury to surrounding tissue. The shape of the gall bladder conforms to the expanded electrode to provide a more even distribution of radiofrequency current to the mucosal layer. Positioning of the balloon electrode and contact with the mucosal layer is visualized and verified by any one of a number of well-known techniques such as fluoroscopy. Such technique is enhanced with the radio-opaque electrolyte solution.

The balloon electrode comprises a thin wall or layer of material, such as well-known latex, having a thickness ranging from 0.005 to 0.0254 cms. (two to ten thousandths of an inch). Since the balloon wall is very thin, the capacitance thereof is thus quite large approximating, for example, 10,000pF. The reactance of this capacitive electrode to, for example, a two megahertz radiofrequency current signal is thus relatively low such as 7 ohms. As a result, the radiofrequency signal is negligibly impeded by the balloon wall, and the balloon is considered to be conductive.

A second lumen 202 included in elongated member 103 transports gall bladder bile from distal end 105 of the catheter to proximal end 115 for drainage therefrom. Depicted in FIG.4 is an enlarged view of the second lumen 202 included in elongated member 103. This drainage occurs external to the balloon as a result of expanding the balloon electrode and consequently forcing the gall bladder bile into the drainage lumen 202.

Positioned about the distal end 105 of the catheter is sensor 109 such as a well-known thermistor for sensing the temperature of the surrounding environment. A volume of this environment includes the current-emitting electrode, the electrolyte solution and the mucosal layer of the gall bladder. Since a direct reading of the mucosal layer temperature is not practically feasible, the temperature of the electrolyte solution and currect-emitting electrode are utilized to closely approximate the temperature of mucosal layer 101. This approximation is derived from a number of experimental measurements along with knowing the power deposition characteristics of the radiofrequency current from the current-emitting electrode as previously described with respect to the spherical model. A third lumen 203 in member 103 of the catheter provides a channel for extending a pair of electrical conductors 110 that are connected to temperature sensor 109.

The electrical conductors from the thermistor are connected to a well-known control circuit 113 included in radiofrequency generator 112 for controlling the amount of current supplied to current-emitting electrode 104 as a function of temperature. The sensed temperature is used to control the amount of current supplied to the current-emitting electrode so as to maintain the temperature of mucosal layer 101 at a temperature such as 50°C to thermally coagulate and destroy that layer of living tissue. Since the power deposition gradient is the steepest at the mucosal layer, the outer layer 122 of the gallbladder is not thermally destroyed since the temperature therein is less than the 42°C temperature necessary to thermally destroy living tissue.

A fourth lumen 204 is also included in elongated member 103 for housing an electrical conductor 111 for interconnecting current-emitting electrode 104 and radiofrequency current generator 112. A large dispersive electrode 120 is placed on the skin 121 of the patient's body (not shown) to receive the radiofrequency current emitted from the balloon electrode and conducted through the body of the patient and to reduce trauma to the body. Another electrical conductor 116 interconnects the dispersive electrode and the radiofrequency current generator.

The method for thermally destroying, or more specifically, thermally coagulating the mucosal layer of the gall bladder includes inserting the distal end of the catheter into body 114 of gall bladder 102 endoscopically by a retrograde route through the duodenum, common bile duct and cystic duct. The capacitive balloon is then fully expanded with the electrolyte solution for making electrical and physical contact with the gall bladder. The balloon when fully expanded does not expand the gall bladder. The balloon catheter also conforms the gall bladder thereto to provide a more uniform distribution of the radiofrequency current from the current-emitting electrode. The use of the electrolyte solution and of the thin wall balloon electrode permits the concentrated deposition of power within the mucosal layer of the gall bladder. This concentrated deposition of power causes the selective heating of the mucosal layer to a thermally destructive temperature without destroying or killing outer layer 122 of the gall bladder. The mucosal layer of the cystic duct is also thermally destroyed to prevent the recurrence of gallstones therein. The temperature of the mucosal layer is maintained at a predetermined temperature such as 50°C for a predetermined period of time to ensure the thermal coagulation and thermal destruction of the entire mucosal layer. Heating times for thermally coagulating and destroying the mucosal layer of the gall bladder in dogs has been derived from experiments in which heating times varied from 6-15 minutes at approximately 50°C for dogs ranging in weight from 8-23kg. The percentage of mucosal layer destroyed varied from 90-100%.

It is to be understood that the above-described apparatus for thermally destroying the layer of an organ is merely an illustrative embodiment of the principles of this invention.

## Claims

1. Apparatus for thermally destroying or coagulating living tissue forming an inner wall in part of the body of a patient, said apparatus comprising an elongated member (100) with an R.F. emitter arrangement (104) at a distal end (105) thereof, a balloon arrangement (106) which when fully extended would conform to the normal contour of the said inner wall, and would serve to position or locate the R.F. emitter arrangement within the inner wall, and a lumen (201) for supplying liquid to the interior of the balloon to expand the balloon, characterized in that a second lumen (202) in direct communication with the exterior of the balloon is provided for withdrawing liquid from the region between the exterior of the balloon and the said inner wall.

2. Apparatus according to claim 1, in which the apparatus is designed to destroy the inner wall of a gall bladder, characterized in that the balloon arrangement (106) is adapted to seal the liquid within said region of the gall bladder whilst the said inner layer is being destroyed.

3. Apparatus according to claim 1 or 2, characterized in that the balloon has a plurality of compartments, and in that means are provided for applying different fluids thereto.

4. Apparatus according to claim 1 or 2, characterized in that a plurality of ports (108) about the distal end of the elongated member communicate with the first mentioned lumen (201) for supplying the liquid into the balloon.

## Patentansprüche

1. Vorrichtung zur thermischen Zerstörung oder Koagulierung von lebendem Gewebe, das eine Innenwand in einem Teil des Körpers eines Patienten bildet, wobei jene Vorrichtung ein längliches Element (100) mit einer HF-Senderanordnung (104) an dessen distalem Ende (105), eine Ballonanordnung (106), die in voll aufgeweitetem Zustand der normalen Kontur jener Innenwand entspricht und zur Positionierung oder Lokalisierung der HF-Senderanordnung in der Innenwand dient, und ein Lumen (201) für die Versorgung des Balloninneren mit Flüssigkeit zur Aufweitung des Ballons aufweist, dadurch gekennzeichnet, daß zur Entfernung von Flüssigkeit aus dem Bereich zwischen dem Ballonäußeren und jener Innenwand ein zweites Lumen (202) in direkter Verbindung mit dem Ballonäußeren vorgesehen ist.

2. Vorrichtung nach Anspruch 1, bei der die Vorrichtung die Innenwand einer Gallenblase zerstören soll, dadurch gekennzeichnet, daß die Ballonanordnung (106) die Flüssigkeit in dem Bereich der Gallenblase einschließen kann, während jene Innenschicht zerstört wird.

3. Vorrichtung nach Anspruch 1 oder 2, dadurch gekennzeichnet, daß der Ballon eine Vielzahl von Abteilungen aufweist und daS Mittel vorgesehen sind, um diese mit verschiedenen Fluiden zu beaufschlagen.

4. Vorrichtung nach Anspruch 1 oder 2, dadurch gekennzeichnet, daß eine Vielzahl von Öffnungen (108) um das distale Ende des länglichen Elementes herum zur Beförderung der Flüssigkeit in den Ballon hinein mit dem erstgenannten Lumen (201) in Verbindung stehen.

**Revendications**

1. Appareil de destruction ou de coagulation thermique de tissus vivants formant une paroi interne dans une partie du corps d'un patient, ledit appareil comprenant un organe allongé (100) avec un arrangement (104) émetteur de R.F. à son extrémité distale (105), un arrangement de ballon (106), qui, une fois complètement gonflé, épouserait le contour normal de ladite paroi interne, et servirait à positionner ou à localiser l'arrangement émetteur de R.F. à l'intérieur de la paroi interne, et une lumière (201) pour fournir du liquide à l'intérieur du ballon pour gonfler le ballon, caractérisé en ce qu'il est prévu une seconde lumière (202) en communication directe avec l'extérieur du ballon pour retirer du liquide de la région entre l'extérieur du ballon et ladite paroi interne.

2. Appareil selon la revendication 1, dans lequel l'appareil est conçu pour détruire la paroi interne d'une vésicule biliaire, caractérisé en ce que l'arrangement de ballon (106) est adapté pour isoler le liquide à l'intérieur de ladite région de la vésicule biliaire tandis que ladite couche interne est détruite.

3. Appareil selon la revendication 1 ou 2, caractérisé en ce que le ballon a une pluralité de compartiments, et en ce que des moyens sont prévus pour leur appliquer différents fluides.

4. Appareil selon la revendication 1 ou 2, caractérisé en ce qu'une pluralité de ports (108) autour de l'extrémité distale de l'organe allongé communiquent avec la première lumière mentionnée (201) pour fournir du liquide dans le ballon.

$F_{IG.}$ 4

$F_{IG.}$ 2

$F_{IG.}$ 1

Fig. 3

Fig. 5